(19) Europäisches Patentamt
European Patent Office
Office européen des brevets

(11) **EP 3 449 823 A1**

(12) **EUROPEAN PATENT APPLICATION**
published in accordance with Art. 153(4) EPC

(43) Date of publication:
**06.03.2019 Bulletin 2019/10**

(51) Int Cl.:
*A61B 5/117* (2016.01)          *A61B 5/11* (2006.01)
*G08B 13/02* (2006.01)          *G08B 25/04* (2006.01)

(21) Application number: **17789699.0**

(22) Date of filing: **27.04.2017**

(86) International application number:
**PCT/JP2017/016867**

(87) International publication number:
**WO 2017/188418 (02.11.2017 Gazette 2017/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **28.04.2016 JP 2016090726**

(71) Applicant: **NEC Solution Innovators, Ltd.
Koto-ku, Tokyo 136-8627 (JP)**

(72) Inventor: **SHIMIZU, Hideyuki
Tokyo 136-8627 (JP)**

(74) Representative: **Vossius & Partner
Patentanwälte Rechtsanwälte mbB
Siebertstrasse 3
81675 München (DE)**

(54) **WALK VIBRATION ANALYZING SYSTEM, VIBRATION ANALYZING DEVICE, WALK VIBRATION ANALYZING METHOD, AND COMPUTER-READABLE RECORDING MEDIUM**

(57)    A walking vibration analysis system 4 includes: a vibration measurement apparatus (1) that measures vibration that occurs accompanying walking of a walker; a vibration analysis apparatus (3) that extracts vibration caused by contact between a heel of the walker and a floor and vibration caused by contact between a toe of the walker and the floor based on the measured vibration, and calculates a characteristic amount unique to the walker based on a result of comparing the extracted former vibration and latter vibration; and an individual specification apparatus (2) that specifies the walker using the calculated characteristic amount and a characteristic amount registered in advance for each user.

Fig.1

WALKING VIBRATION ANALYSIS SYSTEM

VIBRATION MEASUREMENT APPARATUS

VIBRATION ANALYSIS APPARATUS

INDIVIDUAL SPECIFICATION APPARATUS

EP 3 449 823 A1

**Description**

TECHNICAL FIELD

The present invention relates to a walking vibration analysis system, a vibration analysis apparatus, and a walking vibration analysis method for analyzing vibration that occurs due to walking, and furthermore relates to a computer-readable storage medium storing a program for realizing these.

BACKGROUND ART

In recent years, a system in which a sensor is installed in a residence in order to watch over an elderly person when at home, for example, has been proposed. With this kind of system, it is necessary to achieve both the protection of privacy of the resident and identification of the resident. For this reason, in view of the protection of privacy, a method in which the resident is identified using a vibration sensor instead of a camera sensor has been proposed. That is, this is a method of performing identification by extracting a characteristic of an individual from vibration that accompanies walking (hereinafter written as "walking vibration"), which is measured with a vibration sensor installed in a residence.

Specifically, Patent Document 1 discloses a system for specifying an individual. The system disclosed in Patent Document 1 extracts a frequency of vibration that occurs due to an action performed by a person in a space, or a period of multiple steps as a characteristic amount of an individual from the measured walking vibration, compares it with frequencies or periods of individuals collected in advance, and specifies the individual.

Patent Document 2 also discloses a system for specifying an individual. The system disclosed in Patent Document 2 obtains the walking speed and the amplitude peak strength from the measured walking vibration and uses the obtained walking speed and vibration peak strength as the individual information. In general, there is a linear relationship in which if the walking speed increases, the amplitude peak also increases, but the linearity thereof is different depending on the person. The individual specification system disclosed in Patent Document 2 identifies an individual using the fact that the linearity is different depending on the person.

Also, various studies relating to walking have advanced, and for example, Non-Patent Document 1 reports a vibration response property of a floor during walking. Also, Non-Patent Document 2 discloses a study about a walking pattern of a healthy person.

LIST OF PRIOR ART DOCUMENTS

PATENT DOCUMENTS

Patent Document 1: JP 2004-227053A
Patent Document 2: JP 2007-304955A

NON PATENT DOCUMENTS

Non-Patent Document 1: Hideo WATANABE, Akihiko MATSUOKA, Sho KIMURA, Katsuo INOUE, "Vibration Response Characteristics in Residential Floor Finish Structure by Walking - Study of the residential floor vibration characteristics and floor impact sound insulation performance taking footstep sensation into consideration, Part 3", [online], 1998, an article presented by Toda Corporation, [searched on March 30, 2016], Internet <URL: http://www.toda.cojp/lucubration/pdf/p232.pdf>
Non-Patent Document 2: Tetsuo AKIYAMA, Ryo MURATA, Keita MIZUNO, Noboru SEKIYA, "Relationship Between Step Interval, Walking Pattern, and Energy Consumption Amount in a Healthy Person", 2009, Congress of the Japanese Physical Therapy Association

DISCLOSURE OF THE INVENTION

PROBLEM TO BE SOLVED BY THE INVENTION

However, in the system disclosed in Patent Document 1 or Patent Document 2 above, it is necessary to analyze the walking vibration, and therefore there is a problem in that it is not possible to adapt to changes in the installation location of the apparatus that measures the walking vibration, and identification of a certain individual cannot be realized when the installation location of the apparatus is changed. This is because the frequency characteristic of the walking vibration used in Patent Document 1 and the walking speed and linearity of the amplitude peak using in Patent Document 2 change due to the floor material and structure of the building.

Also, Non-Patent Document 1 reports that vibration obtained by simulating walking is applied to multiple floor materials (single flooring, dry double floor, flooring, and the like) and the displacement and frequency responses to the impact force are different depending on the floor material. In other words, due to this, it is understood that data for comparison needs to be collected for each floor material and structure of the building in order to perform reliable individual identification corresponding to changes in the installation location of the apparatus that measures the walking vibration in the method for analyzing the walking vibration. Accordingly, the solution to the problem is not practical due to the fact that a large amount of data is required in order to solve the problems in Patent Document 1 and Patent Document 2 above.

OBJECT OF THE INVENTION

An object of the present invention is to solve the above-described problems and to provide a walking vibration analysis system, a vibration analysis apparatus, a walking vibration analysis method, and a program, according to which it is possible to extract a characteristic amount that is needed to identify an individual from walking vibration without being influenced by a building, such as a floor material and structure of a building.

MEANS FOR SOLVING THE PROBLEM

In order to achieve the above-described object, a walking vibration analysis system according to an aspect of the present invention includes:

a vibration measurement apparatus configured to measure vibration that occurs accompanying walking of a walker;
a vibration analysis apparatus configured to extract vibration caused by contact between a heel of the walker and a floor and vibration caused by contact between a toe of the walker and the floor based on the measured vibration, and calculate a characteristic amount unique to the walker based on a result of comparing the extracted former vibration and latter vibration;
an individual specification apparatus configured to specify the walker using the calculated characteristic amount and a characteristic amount registered in advance for each individual.

In order to achieve the above-described object, a walking vibration analysis apparatus according to an aspect of the present invention extracts vibration caused by contact between a heel of a walker and a floor and vibration caused by contact between a toe of the walker and the floor based on vibration that occurs accompanying walking of the walker, and calculates a characteristic amount unique to the walker based on a result of comparing the extracted former vibration and latter vibration.

Also, in order to achieve the above-described object, a walking vibration analysis method according to an aspect of the present invention includes:

(a) a step of measuring vibration that occurs accompanying walking of a walker;
(b) a step of extracting vibration caused by contact between a heel of the walker and a floor and vibration caused by contact between a toe of the walker and the floor based on the measured vibration, and calculating a characteristic amount unique to the walker based on a result of comparing the extracted former vibration and latter vibration; and
(c) a step of specifying the walker using the calculated characteristic amount and a characteristic amount registered in advance for each individual.

Furthermore, in order to achieve the above-described object, a computer-readable storage medium according to an aspect of the present invention stores a program including commands for causing a computer to execute:

(a) a step of extracting vibration caused by contact between a heel of a walker and a floor and vibration caused by contact between a toe of the walker and the floor based on vibration that occurs accompanying walking of the walker; and
(b) a step of calculating a characteristic amount unique to the walker based on a result of comparing the extracted former vibration and latter vibration.

ADVANTAGEOUS EFFECTS OF THE INVENTION

As described above, according to the present invention, a characteristic amount that is needed for identification of an individual can be extracted from a walking vibration without being influenced by a building, such as a floor material and structure of a building.

BRIEF DESCRIPTION OF THE DRAWINGS

FIG. 1 is a block diagram showing a schematic configuration of a walking vibration analysis system according to an embodiment of the present invention.
FIG. 2 is a block diagram more specifically showing a walking vibration analysis system and a vibration analysis apparatus according to an embodiment of the present invention.
FIG. 3 is a diagram showing an example of acceleration measured in the present embodiment.
FIG. 4 is a diagram showing accelerations for each individual measured in the present embodiment, and FIGS. 4(a) and 4(b) show accelerations measured based on different walkers.
FIG. 5 is a diagram showing an example of a schema for configuring a database in an embodiment of the present invention.
FIG. 6 is a flowchart showing operations of a vibration measurement apparatus and a vibration analysis apparatus according to an embodiment of the present invention.
FIG. 7 is a diagram showing a specific example of vibration start times for heel-floor contact and toe-floor contact, extracted from the acceleration shown in FIG. 5(a).
FIG. 8 is a diagram showing a specific example of vibration start times for heel-floor contact and toe-floor contact, extracted from the acceleration shown in FIG. 5(b).
FIG. 9 is a diagram showing results of determining the start of vibration in the acceleration waveforms shown in FIGS. 5(a) and 5(b), FIG. 9(a) corresponding to FIG. 5(a), and FIG. 9(b) corresponding to FIG. 5(b).
FIG. 10 is a flowchart showing operations of an individual specification apparatus according to an embodiment of the present invention.
FIG. 11 is a diagram showing an example of information stored in a database according to the present embodiment.
FIG. 12 shows a state after updating the information shown in FIG. 11.
FIG. 13 is a block diagram showing a configuration of a modified example of a walking vibration analysis system according to an embodiment of the present invention.
FIG. 14 is a block diagram showing an example of a computer that realizes a vibration measurement apparatus and an individual specification apparatus according to an embodiment of the present invention.

MODE FOR CARRYING OUT THE INVENTION

Embodiment

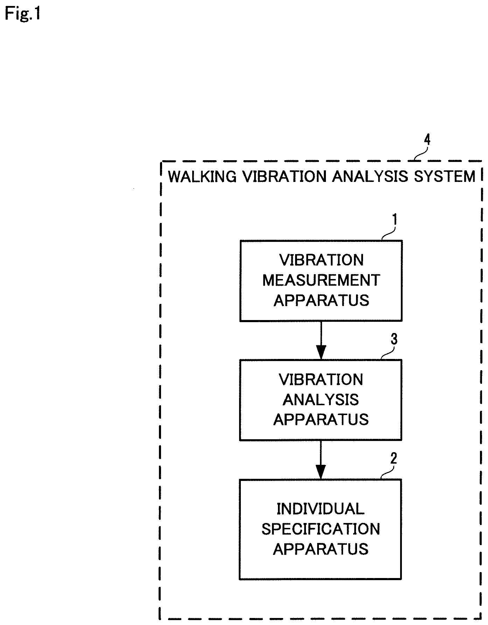

Hereinafter, a walking vibration analysis system, a vibration analysis apparatus, a walking vibration analysis method, and a program according to an embodiment of the present invention will be described with reference to FIGS. 1 to 14.

First, a schematic configuration of a walking vibration analysis system will be described with reference to FIG. 1. FIG. 1 is a block diagram showing a schematic configuration of a walking vibration analysis system according to an embodiment of the present invention.

A walking vibration analysis system 4 according to the present embodiment, which is shown in FIG. 1, is a system for specifying a walker from vibration generated accompanying walking of a walker. As shown in FIG. 1, the walking vibration analysis system 4 includes a vibration measurement apparatus 1, a vibration analysis apparatus 3, and an individual specification apparatus 2.

Among these, the vibration measurement apparatus 1 measures vibration that is generated accompanying walking of a walker. The vibration analysis apparatus 3 extracts vibration caused by contact between the heel of the walker and the floor, and vibration caused by contact between the toe of the walker and the floor, based on the measured vibration. Also, the vibration analysis apparatus 3 calculates a characteristic amount unique to the walker based on a result of comparing the extracted former vibration and latter vibration. Also, the individual specification apparatus 2 specifies the walker using the calculated characteristic amount and characteristic amounts registered for each individual in advance.

Usually, when a walker walks on a floor, identical response characteristics caused by the floor material and structure of a building appear in the vibration caused by contact between the heel and the floor and the vibration caused by contact between the toe of the walker and the floor. In contrast to this, in the present embodiment, the characteristic amount is obtained from a result of comparing the vibration caused by contact between the heel of the walker and the floor and the vibration caused by the toe of the walker and the floor, and therefore the influence of the response characteristic is eliminated from the characteristic amount. For this reason, according to the present embodiment, a characteristic amount that is needed for identifying an individual from the walking vibration can be extracted without being influenced by a building, such as a floor material and a structure of the building.

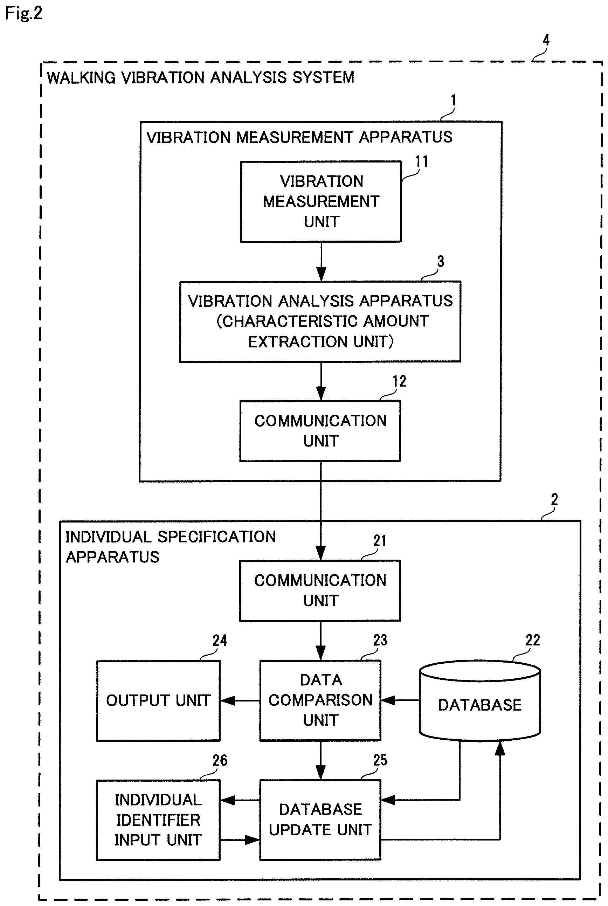

Next, configurations of the walking vibration analysis system 4 and the vibration analysis apparatus 3 according to the present embodiment will be described more specifically with reference to FIG. 2. FIG. 2 is a block diagram more specifically showing a configuration of a walking vibration analysis system according to an embodiment of the present invention.

As shown in FIG. 2, in the present embodiment, the vibration analysis apparatus 3 is incorporated inside of the vibration measurement apparatus 1 and functions as a characteristic amount extraction unit. Accordingly, in the following description, the vibration analysis apparatus 3 is described as a characteristic amount extraction unit 3 in some cases.

Also, in the present embodiment, the vibration measurement apparatus 1 is installed on a floor surface and is connected by wire or wirelessly to the individual specification apparatus 2. The walking vibration analysis system 4 specifies a person who has walked in the periphery of the vibration measurement apparatus 1.

Also, as shown in FIG. 2, the vibration measurement apparatus 1 includes a vibration measurement unit 11, the above-described characteristic amount extraction unit 3, and a communication unit 12.

The vibration measurement unit 11 measures at least one of displacement and acceleration that occur accompanying walking on the floor surface on which the vibration measurement apparatus 1 is installed, as the walking vibration, and performs notification of the walking vibration information for specifying the measured walking vibration to the characteristic amount extraction unit 3.

The vibration measurement unit 11 includes a sensor for detecting vibration. Note that the type of the sensor is not particularly limited, and examples of the sensor include acceleration sensors and displacement sensors, which are commonly in circulation. Also, the walking vibration information is determined according to the type of the sensor included in the vibration measurement unit 11. For example, if the sensor is an acceleration sensor, the acceleration applied to the floor surface is used as the walking vibration information. Also, if the sensor is a displacement sensor, the displacement of the floor surface is used as the walking vibration information. Also, the walking vibration information may be output as a digital signal and may be output as an analog signal.

Here, in the following description, an example will be described in which the vibration measurement unit 11 includes an acceleration sensor that is commonly in circulation. Also, in this example, the walking vibration information is output as a digital signal for specifying the acceleration generated in the floor surface accompanying walking. Specific examples of acceleration sensors include a MEMS acceleration sensor manufactured by Analog Devices Corporation.

In the present embodiment, the characteristic amount extraction unit (vibration analysis apparatus) 3 extracts the vibration caused by contact between the heel of the walker and the floor and the vibration caused by contact between the toe of the walker and the floor from the walking vibration information from the vibration measurement unit 11. Also, by comparing the two, the characteristic amount extraction unit 3 calculates a relative value, such as the difference between the two or the ratio of the two, such that the influence of the response characteristic of the floor material and structure of the building is eliminated, and the result of this calculation is used as the characteristic amount. Also, the characteristic amount extraction unit 3 performs notification of the calculated characteristic amount (hereinafter written as "walking vibration characteristic amount") to the communication unit 12.

Here, examples of the difference between the two include a time difference between the time of starting vibration caused by contact between the heel of the walker and the floor and the time of starting vibration caused by contact between the toe of the walker and the floor. Also, examples of a ratio of the two include an intensity ratio between the amplitude peak of vibration caused by the contact between the heel of the walker and the floor and the amplitude peak of vibration caused by the contact between the toe of the walker and the floor. Note that in the following description, the time difference between the time of starting vibration caused by the contact between the heel and the floor and the time of starting vibration caused by the contact between the toe and the floor is used as the walking vibration characteristic amount.

Also, the above-described Non-Patent Document 2 reports that the distance between the heel and the toe does not change radically, and furthermore, that even when the walking speed changes, the walking speed is adjusted by a factor other than the distance between the heel and toe (step width and pace). Accordingly, it can be expected that the walking vibration characteristic amount used in the present embodiment is a characteristic amount that changes little. Also, in the present embodiment, measurement error and chronological change are reflected in the system with comparison processing performed by a later-described data comparison unit 23 of the individual specification apparatus 2 using the error information and update processing of data in the data update unit 25.

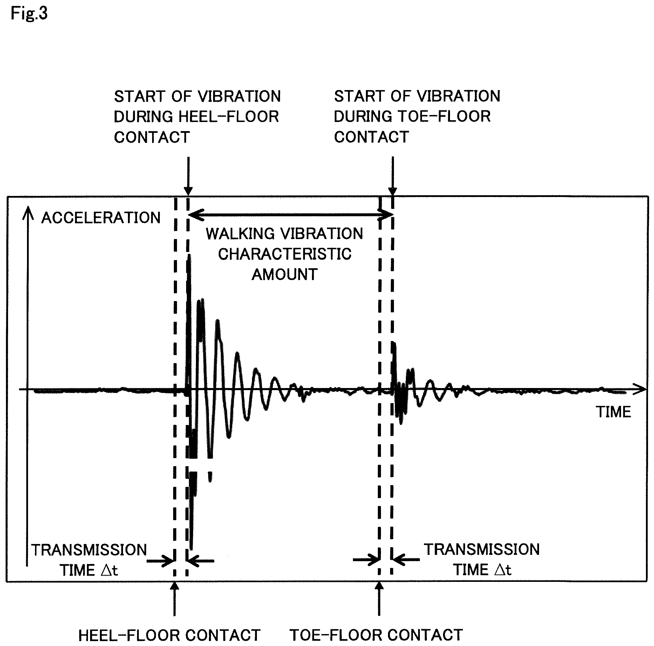

FIG. 3 is a diagram showing an example of acceleration measured in the present embodiment. FIG. 3 shows a typical example of acceleration in the walking vibration measured by the vibration measurement unit 11. Also, FIG. 3 shows two prominent vibrations, namely vibration caused by contact between the heel and the floor (hereinafter also written as "heel-floor contact") and vibration caused by contact between the toe and the floor (hereinafter also written as "toe-floor contact"). The prominent vibration on the left side of the drawing is vibration at the time of heel-floor contact, and the prominent vibration on the right side of the drawing is vibration at the time of toe-floor contact.

Furthermore, in FIG. 3, the timings of heel-floor contact, the start of vibration at the time of heel-floor contact, toe-floor contact, and the start of vibration at the time of toe-floor contact are displayed schematically. Also, as shown in FIG. 3, the transmission time from when the force is applied to the floor material to when the vibration is transmitted to the acceleration sensor depends on the floor material, and when the transmission time is set as $\Delta t$, the following Equations 1 and 2 hold true.

Equation 1

(Vibration start time of heel-floor contact) = (Heel-floor contact time) + $\Delta t$

Equation 2

(Vibration start time of toe-floor contact) = (Toe-floor contact time) + $\Delta t$

As in the present embodiment, when the walking vibration characteristic amount is set as the time difference between the starts of vibration of the heel-floor contact and the toe-floor contact, the walking vibration characteristic amount can be calculated using the following Equation 3.

Equation 3

(Walking vibration characteristic amount) = (Vibration start time of toe-floor contact)

− (vibration start time of heel-floor contact)

= (Toe-floor contact time) − (heel-floor contact time)

Accordingly, the walking vibration characteristic amount is a relative value obtained by eliminating $\Delta t$, which is influenced by the floor material, and is a characteristic value that depends only on the walking characteristic of the individual.

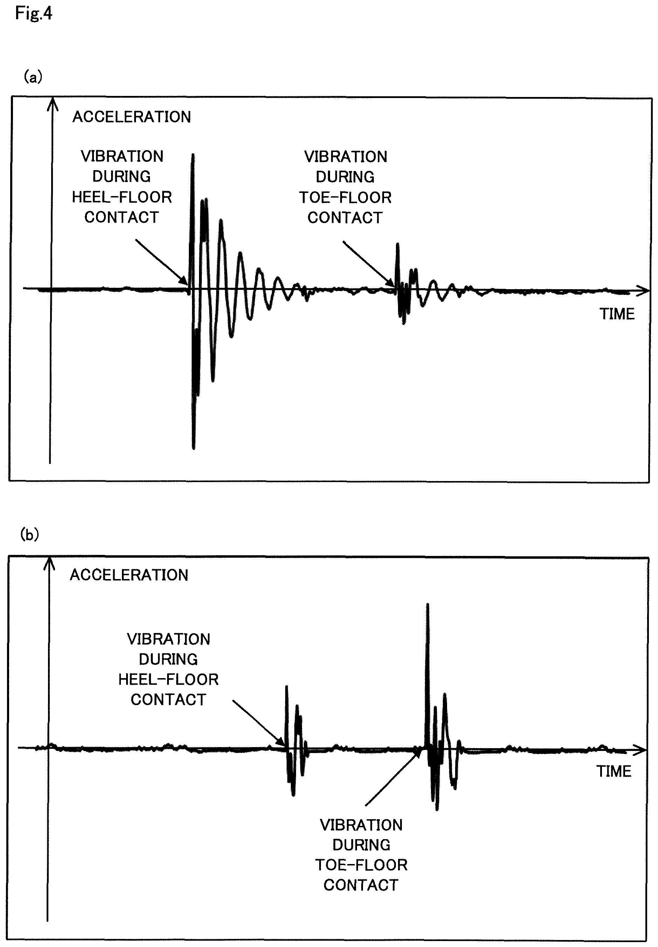

Also, FIG. 4 is a diagram showing accelerations for each individual measured in the present embodiment, FIGS. 4(a) and 4(b) showing accelerations measured from different walkers. As shown in FIGS. 4(a) and 4(b), even if the individuals are different, the waveforms of the accelerations are similar, and two prominent vibrations can be seen in each waveform.

As described above, the characteristic amount extraction unit 3 extracts the vibration start point of the heel-floor contact and the vibration start point of the toe-floor contact from the acceleration shown in FIGS. 3, 4(a), 4(b), and the like. The extraction of the vibration start points can be performed by determining whether the floor state is vibrating or still, using the variance value of the acceleration values.

Specifically, for example, the acceleration sensor has an output error that conforms to a normal distribution with a deviation A. In this case, it can be expected that the variance value of the acceleration values measured when the floor is still falls within a multiple of $A^2$ at most. The upper limit value in the still state is defined as a still-time threshold. Accordingly, each time the acceleration is newly transferred from the vibration measurement unit 11, the characteristic amount extraction unit 3 calculates a variance value according to the value of the transferred acceleration and multiple prior values of acceleration. Also, if the variance value is not less than the still-time threshold, the characteristic amount extraction unit 3 determines that the floor surface is vibrating, and furthermore, if the variance value is less than the still-time threshold value after determining that the floor surface is vibrating, the characteristic amount extraction unit 3 determines that the floor surface is still. Through this kind of processing, the vibration start point of heel-floor contact and the vibration start point of toe-floor contact are extracted.

The communication unit 12 transmits the walking vibration characteristic amount calculated by the characteristic amount extraction unit 3 to the individual specification apparatus 2. Also, in the present embodiment, the method of communication performed by the communication unit 12 may be by wire or wireless. Furthermore, the communication protocol that is used may be an existing communication protocol or a unique communication protocol.

Also, as shown in FIG. 2, in the present embodiment, the individual specification apparatus 2 includes a communication unit 21, a database 22, a data comparison unit 23, an output unit 24, a database update unit 25, and an individual identifier input unit 26.

The communication unit 21 receives the walking vibration characteristic amount from the vibration measurement apparatus 1 and sends the received walking vibration characteristic amount to the data comparison unit 23.

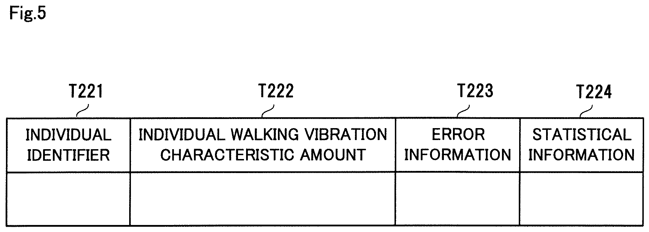

For each user, in advance, the database 22 registers information that is necessary for specifying an individual from the walking vibration characteristic amount, that is, the walking vibration characteristic amount unique to the individual and the allowable range of error. Specifically, the database 22 is configured using the following schema, which is shown in FIG. 5. FIG. 5 is a diagram showing an example of a schema configuring a database in an embodiment of the present invention.

As shown in FIG. 5, the database 22 includes individual identifier T221, individual walking vibration characteristic amount T222, error information T223, and statistical information T224 as fields, and stores information of an individual in each field.

Among these, individual identifier T221 stores information uniquely specifying an individual, such as an ID or user name. Individual walking vibration characteristic amount T222 stores walking vibration characteristic amounts measured for each individual. Error information T223 stores the error in the individual walking vibration characteristic amount, such as a variance value obtained when the individual walking vibration characteristic amount is derived from multiple samples. Statistical information T224 stores statistical information that is needed for updating the walking vibration characteristic amount and the error information, such as a sample count for when the individual walking vibration characteristic amount is derived from multiple samples.

Also, the format of the database 22 is not particularly limited, and any format, such as a hierarchical format, a network format, or a relational format, may be used there as. Furthermore, each field may include multiple elements. For example, error values that differ according to the range of values of the walking vibration characteristic amount may be stored in the error information. The sample counts, as well as sum totals or squared sum totals, or the like, may be stored in the statistical information.

Note that in the following description, it is assumed that the user name is used as the individual identifier. Also, it is assumed that the average value of multiple samples for the time difference between the start time of vibration caused by heel-floor contact and the start time of vibration caused by toe-floor contact as the individual walking vibration characteristic amount. Furthermore, it is assumed that the variance value of the individual walking vibration characteristic amount is used as the error information and the sample count and squared sum total of the individual walking vibration characteristic amounts are used as the statistical information.

The data comparison unit 23 compares the walking vibration characteristic amount sent from the communication unit 21 with the individual walking vibration characteristic amount and the error information stored in the database 22. Also, if there is a match as a result of the comparison, the database comparison unit 23 specifies the walker using the individual identifier that corresponds to the matched walking vibration characteristic amount. On the other hand, if there is no match, the data comparison unit 23 determines that the comparison result is not applicable.

Specifically, the data comparison unit 23 obtains a difference between the walking vibration characteristic amount sent from the communication unit 21 and the individual walking vibration characteristic amount stored in the database 22, and determines whether or not the obtained difference falls within the range of the variance value (error).

If it is determined that the difference falls within the range of the variance value, the data comparison unit 23 uses the corresponding user ID as the comparison result. In this case, the data comparison unit 23 sends the comparison result to the output unit 24. Also, the data comparison unit 23 sends the comparison result and the walking vibration characteristic amount to the database update unit 25.

On the other hand, if it is determined that the difference does not fall within the range of the variance value, the data comparison unit 23 determines that the comparison result is not applicable. In this case as well, the data comparison unit 23 sends the comparison result and the walking vibration characteristic amount to the database update unit 25.

The output unit 24 outputs the comparison result from the data comparison unit 23. The output destination of output performed by the output unit 24 is not particularly limited, and may be a display apparatus such as a liquid crystal display, or an external terminal apparatus connected by a network or the like. Also, in the latter case, the connection with the terminal apparatus may be wired or wireless, and the communication protocol used for the communication of the terminal apparatus may be an existing communication protocol or a unique communication protocol.

When the database update unit 25 receives the comparison result and the walking vibration characteristic amount sent from the data comparison unit 23, the database update unit 25 updates the database 22 using the comparison result and the walking vibration characteristic amount. Specifically, if the walker has been specified by the data comparison unit 23, the database 22 is updated through statistical processing using the measured walking vibration characteristic amount.

Also, the updating in this case means updating the values for the individual walking vibration characteristic amount, the error information, and the statistical information, which are registered in the database 22 for the walker who was specified this time, through re-calculation with the measured walking vibration characteristic amounts added.

Specifically, it is assumed that the walking vibration characteristic amounts of N instances prior to the present instance of specification were obtained for the walker who was specified in the present instance. In this case, the individual walking vibration characteristic amount, the error information, and the statistical information of the person registered in the database 22 are indicated as f(N) using a common sample count N. Also, since the walking vibration characteristic amount of the N+1-th instance is measured in the current instance, the sample count is increased to N+1. The walking vibration characteristic amount, the error information, and the statistical information are set to f(N+1) and registered in the database 22 using a similar function.

Also, in the present embodiment, for example, the average value of the multiple samples is used as the individual walking vibration characteristic amount, and therefore the walking vibration characteristic amount is indicated as f(N)=(sum total of N samples)/N. The error information and the statistical information are similarly indicated by equations in which the sample count N is used.

On the other hand, if no walker was specified by the data comparison unit 23, the database update unit 25 requests output of an individual identifier to the individual identifier input unit 26 in order to newly register the individual walking vibration characteristic amount. When the individual identifier input unit 26 outputs the individual identifier in response to the request, the data comparison unit 25 receives the individual identifier and registers the received individual identifier and the new walking vibration characteristic amount in the database 22.

When the output of the individual identifier is requested by the database update unit 25 as described above, the individual identifier input unit 26 prompts input of the individual identifier to an external manager or the like. Then, when the individual identifier is input, the individual identifier input unit 26 transfers the input individual identifier to the database update unit 25. The means for prompting input of the individual identifier to the outside and the means for causing input of the individual identifier is not particularly limited. Examples of these means include an apparatus such as an interactive personal computer, and an external terminal apparatus that holds individual identifiers.

Description of Operation

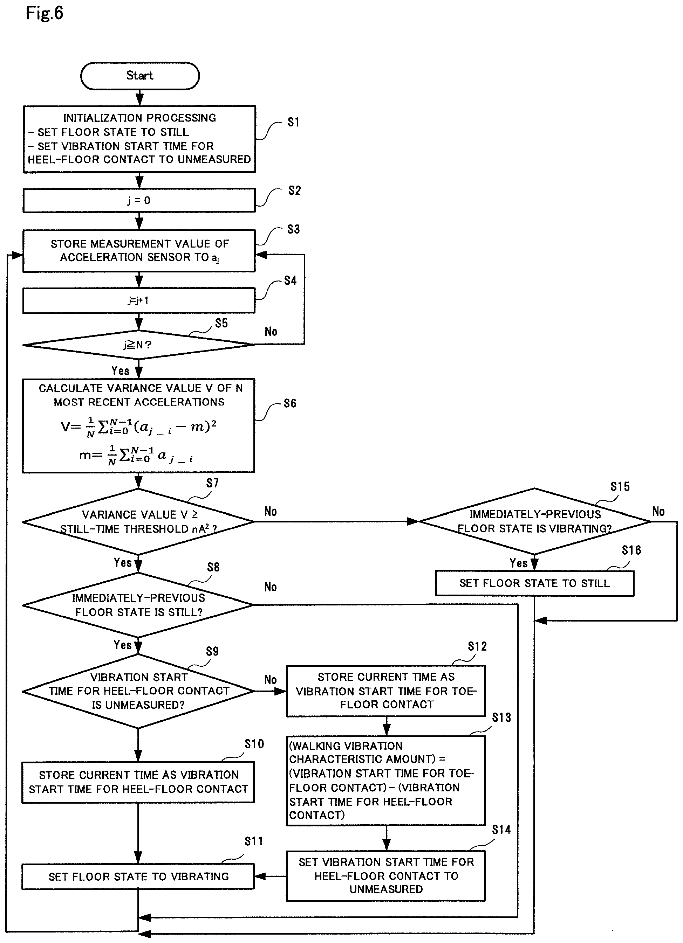

Next, operations of the walking vibration analysis system 4 according to an embodiment of the present embodiment will be described with reference to FIGS. 6 to 12. Also, in the present embodiment, the walking vibration analysis method is implemented by causing the walking vibration analysis system 4 to operate. Accordingly, the description of the walking vibration analysis method of the present embodiment is substituted with the following description of the operation of the walking vibration analysis system 4. Also, in the following description, FIGS. 1 to 4 are referenced as appropriate.

First, the operations of the vibration measurement apparatus 1 and the vibration analysis apparatus 3 will be described with reference to FIG. 6. FIG. 6 is a flow chart showing operations of the vibration measurement apparatus and the vibration analysis apparatus according to the embodiment of the present invention. First, it is presupposed that in the vibration measurement apparatus 1, the vibration measurement unit 11 outputs the measurement values (walking vibration information) of the acceleration sensor in a setting interval. In this state, the processing shown in FIG. 6 is performed.

As shown in FIG. 6, first, the characteristic amount extraction unit 3 executes initialization processing (step S1). Specifically, the characteristic amount extraction unit 3 uses n times $A^2$ as the operation threshold, and N as the measurement point count to be used in the calculation of the variance value, and in the initial state, the floor surface is still, with no walking vibration being applied. Note that n is a real number larger than 0 that is obtained in advance, and N is a positive number that is determined in advance.

Next, the characteristic amount extraction unit 3 sets the value of a variable j to 0 (step S2). Then, each time the vibration measurement unit 11 newly measures acceleration and outputs a measurement value, the characteristic amount extraction unit 3 stores the j-th measurement value $a_j$ (step S3), increases the value of variable j by 1 (step S4), and determines whether or not the value of variable j is greater than or equal to N (step S5).

If it is determined in step S5 that the value of the variable j is greater than or equal to N, the characteristic amount extraction unit 3 once again executes step S3 at a timing at which the measurement value is output. On the other hand, if it is determined in step S5 that the value of the variable j is greater than or equal to N, N or more measurement points used in the calculation of the variance value are accumulated, and the calculation of the variance value becomes possible, and therefore the characteristic amount extraction unit 3 calculates an average value m and a variance value V of acceleration (measurement value) using the following Equations 4 and 5 (step S6).

Equation 4

$$m = \frac{1}{N} \sum_{i=0}^{N-1} a_{j-1}$$

Equation 5

$$V = \frac{1}{N} \sum_{i=0}^{N-1} (a_{j-1} - m)^2$$

Next, the characteristic amount extraction unit 3 determines whether or not the variance value V is greater than or equal to the still-time threshold $nA^2$ (step S7). If it is determined in step S7 that the variance value V is greater than or equal to the still-time threshold value $nA^2$, the characteristic amount extraction unit 3 further determines whether or not the immediately-previous floor state is still (step S8).

If it is determined in step S8 that the immediately-previous floor state is not still, no change in the floor state has occurred, and therefore the characteristic amount extraction unit 3 once again executes step S3. On the other hand, if it is determined in step S8 that the immediately-previous floor state is still, the characteristic amount extraction unit 3 determines whether or not the time of the vibration start point of the heel-floor contact has not been measured (step S9).

If it is determined in step S9 that the time of starting vibration in the heel-floor contact has not been measured, it thought that the vibration that has newly occurred is due to heel-floor contact, and the characteristic amount extraction unit 3 stores the current time as the time of starting vibration of the heel-floor contact (step S10). Next, in this case, it is considered that vibration has newly occurred due to heel-floor contact, and the characteristic amount extraction unit 3 determines that the floor state is vibrating (step S11).

On the other hand, if it is determined in step S9 that the time of the vibration start point of heel-floor contact is not unmeasured (if the time of starting vibration of heel-floor contact has been measured), it is thought that the newly-occurring vibration is caused by toe-floor contact, and the characteristic amount extraction unit 3 stores the current time as a time of starting vibration of toe-floor contact (step S12).

Next, after executing step S12, the times of starting vibration for heel-floor contact and toe-floor contact have been acquired, and therefore the characteristic amount extraction unit 3 calculates the difference between the two and stores the result as the walking vibration characteristic amount (step S13).

Next, after extracting the walking vibration characteristic amount in step S13, the characteristic amount extraction unit 3 once again stores the time of starting vibration of heel-floor contact, and therefore sets the vibration start time to unmeasured (step S14). Thereafter, in this case, the vibration is considered to have newly occurred due to toe-floor contact, and the characteristic amount extraction unit 3 determines that the floor state is vibrating (step S11).

Also, if it is determined in step S7 above that the variance value V is less than the still threshold $nA^2$, the characteristic amount extraction unit 3 further determines whether or not the immediately-previous floor state is vibrating (step S 15).

If it is determined in step S15 that the immediately-previous floor state is not vibrating, no change in the floor state has occurred, and therefore the characteristic amount extraction unit 3 once again executes step S3. On the other hand, if it is determined in step S15 that the immediately-previous floor state is vibrating, the vibration caused by one of heel-floor contact or toe-floor contact is considered to have ended, and thus the characteristic amount extraction unit 3 sets the floor state to still (step S16).

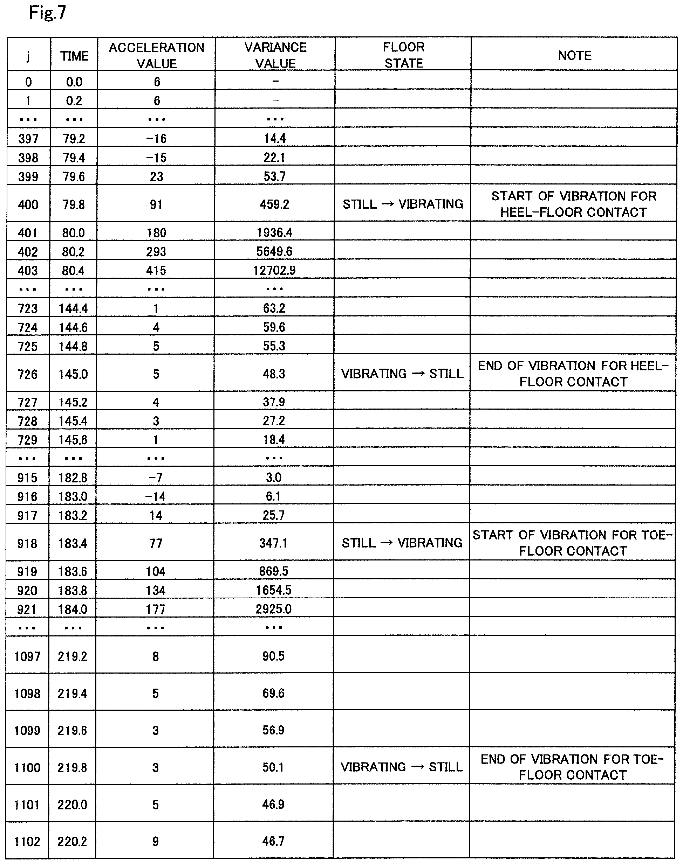

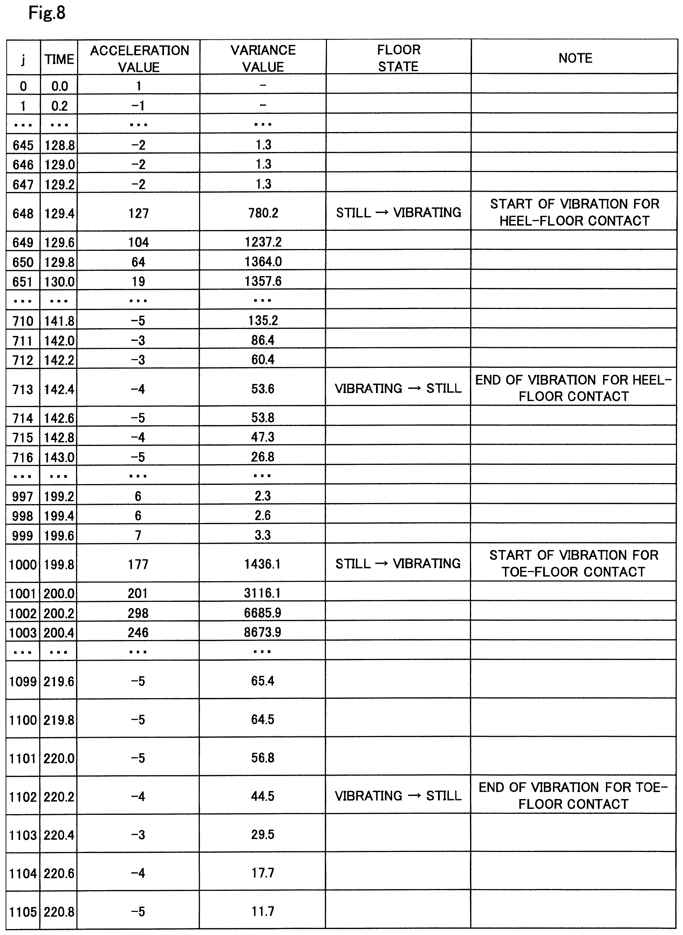

Next, a specific example of the extracted times of starting vibration will be described with reference to FIGS. 7 and 8. FIG. 7 is a diagram showing a specific example of vibration start times for heel-floor contact and toe-floor contact extracted from the acceleration shown in FIG. 5(a). FIG. 8 is a diagram showing a specific example of vibration start times for heel-floor contact and toe-floor contact extracted from the acceleration shown in FIG. 5(b).

Also, in FIGS. 7 and 8, j indicates the order in which the acceleration values (measurement values) were measured. "Time" indicates the amount of time that has elapsed in units of milliseconds, with the time of measuring the acceleration value in which j=0 set to zero.

In the example of FIG. 7, the deviation A of the acceleration sensor is set to A=3, the still-time threshold is set to be 6 (n=6) times $A^2$, which is 54, and the measurement point count N used to calculate the variance value is set to 20. The variance column is constituted by variance values of 20 measurement points' worth of acceleration, which include the 19 immediately-previous measurement points.

In this case, following the flow shown in FIG. 6, the floor state transitions from still to vibrating at j=400, from vibrating to still at j=726, from still to vibrating at j=918, and from vibrating to still at j=1100.

Among these, j=400 is determined as the start of vibration for heel-floor contact, and the vibration start time for heel-floor contact is 79.8 milliseconds. j=918 is determined as the start of vibration for toe-floor contact, and the vibration start time for toe-floor contact is 183.4 milliseconds. Accordingly, the time difference between the start of vibration for heel-floor contact and for toe-floor contact, which is the walking vibration characteristic amount, is 103.6 milliseconds.

Also, in the example of FIG. 8, similarly to the example of FIG. 7, following the flow shown in FIG. 6, the vibration start time for heel-floor contact is 129.4 milliseconds and the vibration start time for toe-floor contact is 199.8 milliseconds. Accordingly, the time difference between the start of vibration for heel-floor contact and for toe-floor contact, which is the walking vibration characteristic amount, is 70.4 milliseconds.

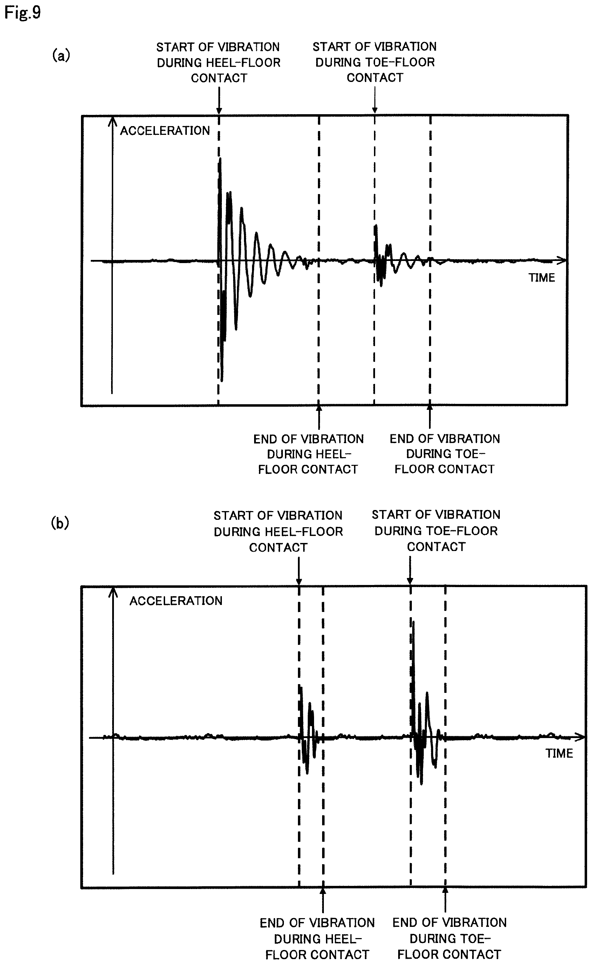

FIG. 9 is a diagram showing the results of determining the vibration start times in the acceleration waveforms shown in FIGS. 5(a) and 5(b), FIG. 9(a) corresponding to FIG. 5(a), and FIG. 9(b) corresponding to FIG. 5(b). Specifically, FIGS. 9(a) and 9(b) indicate the locations of the starts and ends of vibration for heel-floor contact and toe-floor contact, which were specified through applying the flow shown in FIG. 6.

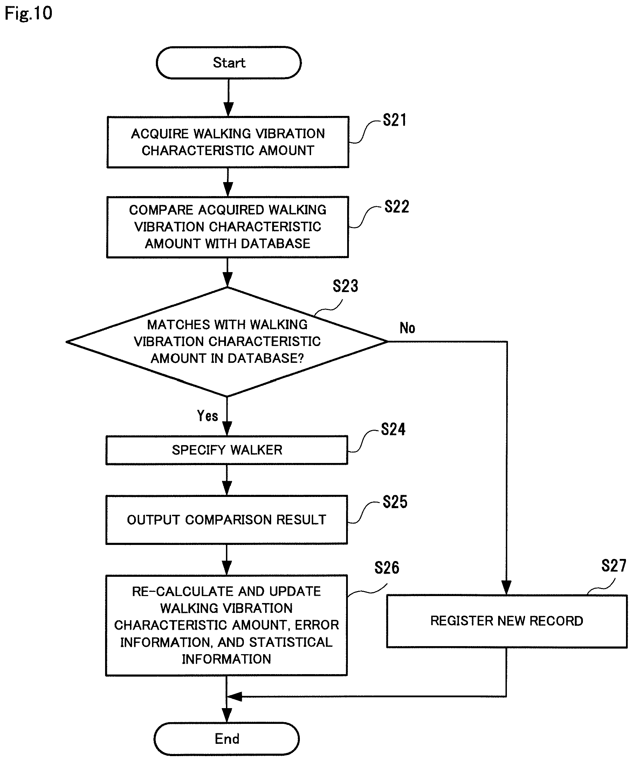

Next, the operation of the individual specification apparatus 2 will be described with reference to FIG. 10. FIG. 10 is a flowchart showing operations of an individual specification apparatus according to an embodiment of the present invention. The operation of the individual specification apparatus 2 shown in FIG. 10 is started when the walking vibration characteristic amount is sent from the vibration measurement apparatus 1 to the individual specification apparatus 2.

As shown in FIG. 10, the communication unit 21 acquires the walking vibration characteristic amount from the vibration measurement apparatus 1 and sends the acquired walking vibration characteristic amount to the data comparison unit 23 (step S21).

Next, the data comparison unit 23 compares the walking vibration characteristic amount acquired in step S21 with the individual walking vibration characteristic amounts and error information stored in the database 22 (step S22).

Next, based on the result of the comparison, the data comparison unit 23 determines whether or not the walking vibration characteristic amount acquired in step S21 matches any of the individual walking vibration characteristic amounts stored in the database 22 (step S23).

If it is determined in step S23 that the walking vibration characteristic amount acquired in step S21 matches the walking vibration characteristic amount of an individual stored in the database 22, the data comparison unit 23 specifies the walker using the individual information corresponding to the matching walking vibration characteristic amount (step S24).

Also, in step S24, the data comparison unit 23 sends the corresponding user ID as the comparison result to the output unit 24. Accordingly, the output unit 24 outputs the comparison result from the data comparison unit 23 (step S25). Also, the data comparison unit 23 sends the comparison result and the walking vibration characteristic amount to the database update unit 25.

Next, when step S25 is executed, the database update unit 25 receives the comparison result and the walking vibration characteristic amount sent from the database comparison unit 23 and updates the database 22 using the comparison result and the walking vibration characteristic amount (step S26). Specifically, through statistical processing using the measured walking vibration characteristic amount, the database update unit 25 re-calculates the individual walking vibration characteristic amount, the error information, and the statistical information registered in the database 22 in advance. Then, the database update unit 25 updates the database 22 with the obtained values.

On the other hand, if it is determined in step S23 that the walking vibration characteristic amount acquired in step S21 does not match the individual walking vibration characteristic amount stored in the database 22, the data comparison unit 23 determines that the comparison result is not applicable. In this case as well, the data comparison unit 23 sends the comparison result and the walking vibration characteristic amount to the database update unit 25.

Thereafter, step S27 is executed. In step S27, the database update unit 25 receives the comparison result and the walking vibration characteristic amount sent from the database comparison unit 23 and updates the database 22 using the comparison result and the walking vibration characteristic amount. Specifically, the database update unit 25 requests output of the individual identifier to the individual identifier input unit 26 and registers the output individual identifier and the new walking vibration characteristic amount obtained in step S21 in association with each other in the database 22.

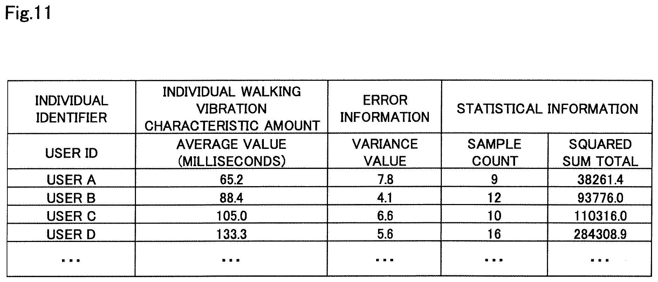

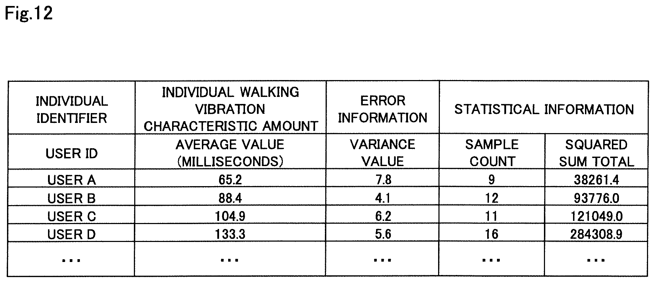

Here, steps S21 to S26 above will be described with reference to FIGS. 11 and 12. FIG. 11 is a diagram showing an example of information stored in a database according to the present embodiment. FIG. 12 shows a state after updating the data shown in FIG. 11.

As shown in FIG. 11, the database 22 stores user IDs as individual identifiers, and stores the average values of multiple samples of time differences between the start of vibration for heel-floor contact and the start of vibration for toe-floor contact as the individual walking vibration characteristic amounts. Also, the database 22 stores the variance values of the individual walking vibration characteristic amounts as the error information and stores the sample counts and squared sum totals of the individual walking vibration characteristic amounts as the statistical information.

The data comparison unit 23 determines whether or not the walking vibration characteristic amounts received from the vibration measurement apparatus 1 and the individual walking vibration characteristic amounts stored in the database 22 match within the range of the error.

Also, if it is assumed that the acceleration shown in FIG. 4(a) above is measured and the walking vibration characteristic amount is 103.6 milliseconds, the walking vibration characteristic amount is determined as matching the walking vibration characteristic amount 105 milliseconds of user C in FIG. 11 within the range of the error 6.6 milliseconds. Also, if it is assumed that the acceleration shown in the example of FIG. 4(b) above is measured and the walking vibration characteristic amount is 70.4 milliseconds, the walking vibration characteristic amount is determined as matching the walking vibration characteristic amount 65 of user A within the range of the error 7.8 milliseconds. Accordingly, the user C and the user A are the respective comparison results for the walking vibrations shown in FIGS. 4(a) and 4(b). Thereafter, the output unit 24 outputs the comparison result output by the data comparison unit 23.

Also, if an individual was specified as a result of the comparison performed by the data comparison unit 23, the database update unit 25 updates the database 22 using the walking vibration characteristic amount. Here, the information accumulated in the database 22 is the sample count N, the individual walking vibration characteristic amount $T_N$, a variance value $V_N$ of the individual walking vibration characteristic amount, and a squared sum total $S_N$. The data comparison unit 23 updates these pieces of information using the information of the individual specified by the above-described processing.

Also, the walking vibration characteristic amount from the vibration measurement apparatus 1 used for comparison is set as T. When the database 22 is updated, the database update unit 25 uses a commonly-known method for sequentially calculating average values and variance values. First, the database update unit 25 updates the sample count to N+1 and the squared sum total to $S_{N+1}=S_N+T^2$. Next, the database update unit 25 updates the individual walking vibration characteristic amount to $T_{N+1}=(N\times T_N+T)/(N+1)$.

Finally, the database update unit 25 updates the variance value using $V_{N+1}=(S_{N+1}-(N+1)\times T_{N+1}^2)/(N+1)$. In this manner, the most recent walking vibration characteristic amount is reflected in the database 22.

With the example of FIG. 4(a) above, the comparison result is the user C, and therefore in FIG. 11, the sample count N = 10, the walking vibration characteristic amount $T_N$ = 105.0 milliseconds, the variance value $V_N$ of the individual walking vibration characteristic amount = 6.6, and the squared sum total $S_N$ = 110316.0. Also, the walking vibration characteristic amount from the vibration measurement apparatus 1 satisfies T = 103.6 milliseconds.

When these values are used, as shown in FIG. 11, the updated values are calculated such that the sample count N+1 = 11, the walking vibration characteristic amount $T_{N+1}$ = 104.9 milliseconds, the variance value $V_{N+1}$ of the individual walking vibration characteristic amount = 6.2, and the squared sum total $S_{N+1}$ = 121049.0.

If the result of the comparison performed by the data comparison unit 23 is not applicable, the database update unit 25 requests a newly-associated user ID to the individual identifier input unit 26. In this case, the individual identifier input unit 26 prompts external input of a user ID, and upon obtaining a user ID, the individual identifier input unit 26 transfers the user ID to the database update unit 25.

If an individual was not specified as a result of the comparison performed by the data comparison unit 23, the database update unit 25 registers the user ID of the individual identifier input unit 26 and the walking vibration characteristic amount of the vibration measurement apparatus 1 in the database 22. Note that in this registration, the sample count is 1, and therefore the initial value for the variance value is a fixed value or a value that is set with reference to the variance value of another user.

As described above, according to the present embodiment, a characteristic amount that is needed for identification of an individual can be extracted from a walking vibration without being influenced by a building, such as a floor material and structure of a building. Also, a newly-calculated characteristic amount is added, and the registered characteristic amount of an individual is re-calculated, and therefore it is possible to track changes in an individual, such as aging and health. Furthermore, in order to prevent being influenced by a building, such as the floor material and structure of a building, it is not necessary to increase the number of sensors, and therefore an increase in cost caused by an increase in the number of sensors is also suppressed.

Modified Example

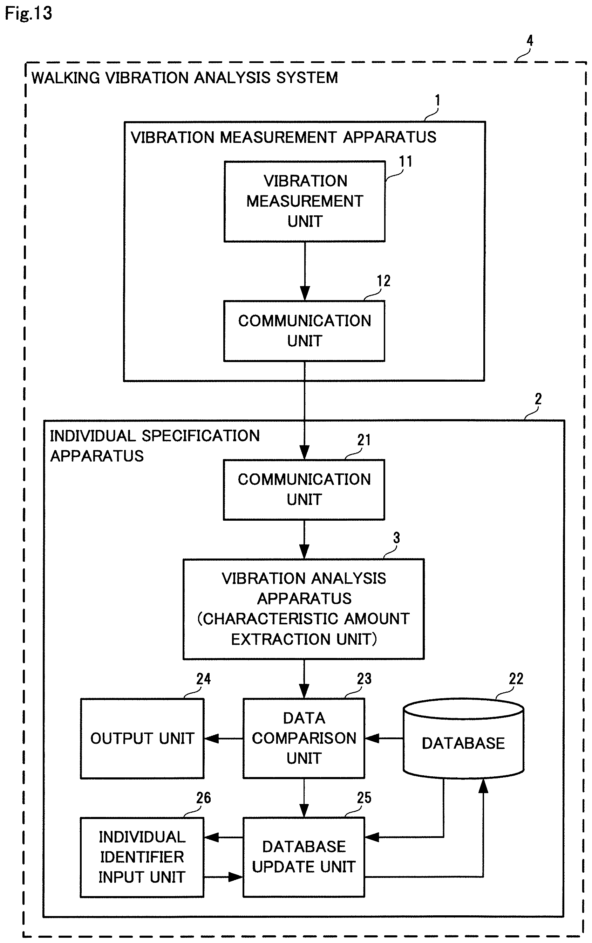

Next, a modified example of the walking vibration analysis system according to the embodiment of the present invention will be described with reference to FIG. 13. FIG. 13 is a block diagram showing a configuration of the modified example of the walking vibration analysis system according to the embodiment of the present invention.

In the example shown in FIG. 2, the vibration analysis apparatus 3 is incorporated in the vibration measurement apparatus 1 and functions as the characteristic amount extraction unit 3 in the vibration measurement apparatus 1. However, the vibration analysis apparatus 3 does not necessarily need to be incorporated in the vibration measurement apparatus 1 and may be incorporated in the individual specification apparatus 2 as long as the walking vibration information can be obtained as an input.

FIG. 13 shows an example in which the vibration measurement apparatus (characteristic amount extraction unit) 3 is incorporated in the individual specification apparatus 2. In the case of using the configuration shown in FIG. 13, the vibration measurement apparatus 1 includes only the vibration measurement unit 11 and the communication unit 12. The communication unit 12 transmits the walking vibration information measured by the vibration measurement unit 11 to the individual specification apparatus 2.

Also, as shown in FIG. 13, unlike the example shown in FIG. 2, the individual specification apparatus 2 newly includes the characteristic amount extraction unit 3. For this reason, upon receiving the walking vibration information, the communication unit 21 transfers the received walking vibration information to the characteristic amount extraction unit 3. Similarly to the example of FIG. 2, the characteristic amount extraction unit 3 calculates the walking vibration characteristic amount in accordance with the flow shown in FIG. 6. Thereafter, the characteristic amount extraction unit 3 transfers the calculated walking vibration characteristic amount to the data comparison unit 23. Upon receiving the walking vibration characteristic amount, steps S21 to S26 shown in FIG. 10 are executed by the data comparison unit 23, the data update unit 25, and the individual identification input unit 26.

According to the present modified example, only the vibration measurement unit 11 and the communication unit 12 need be mounted in the vibration measurement apparatus 1, and therefore general-purpose sensors that can measure vibration (an acceleration sensor, a displacement sensor, etc.) can be used as the vibration measurement apparatus 1 without adding a special installation. That is, according to the present modified example, the cost relating to the structure of the vibration measurement apparatus can be reduced. Also, since the flow for extracting the walking vibration characteristic amount shown in FIG. 6 is concentrated in the individual specification apparatus 2, maintainability increases, and the cost related to maintenance can also be reduced.

Program

A first program according to the present embodiment need only be a program that causes a computer to execute steps S1 to S16 shown in FIG. 6. The vibration analysis apparatus 3 according to the present embodiment can be realized by installing this program in a computer and executing it. In this case, the CPU (Central Processing Unit) of the computer functions as the vibration analysis apparatus 3 and performs processing.

Also, a second program according to the present embodiment need only be a program that causes a computer to execute steps S21 to S27 shown in FIG. 10. The individual specification apparatus 2 according to the present embodiment can be realized by installing this program in a computer and executing it. In this case, the CPU (Central Processing Unit) of the computer functions as the communication unit 21, the data comparison unit 23, the database update unit 25, and the individual identifier input unit 26, and performs processing.

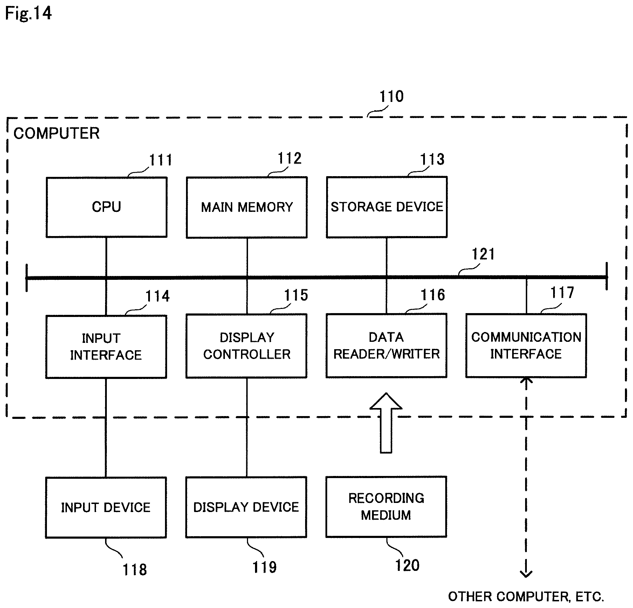

Here, an example of a computer that can execute the first and second programs according to the present embodiment will be described with reference to FIG. 14. FIG. 14 is a block diagram showing an example of a computer that realizes a vibration measurement apparatus and an individual specification apparatus according to an embodiment of the present invention.

As shown in FIG. 14, the computer 110 includes a CPU 111, a main memory 112, a storage apparatus 113, an input interface 114, a display controller 115, a data reader/writer 116, and a communication interface 117. These units are connected via a bus 121 so as to be able to perform data communication with each other.

The CPU 111 loads the programs (codes) according to the present embodiment, which is stored in a storage apparatus 113, to the main memory 112, and implements various types of calculations by executing the programs in a predetermined sequence. The main memory 112 is typically a volatile storage apparatus, such as a DRAM (Dynamic Random Access Memory). Also, the program according to the present embodiment is provided in a state of being stored in a computer-readable storage medium 120. Note that the programs according to the present embodiment may be distributed over the Internet, which is connected to via the communication interface 117.

Also, specific examples of the storage apparatus 113 include a hard disk drive, as well as a semiconductor storage apparatus such as a flash memory. The input interface 114 mediates data transfer between the CPU 111 and an input device 118 such as a keyboard and mouse. The display controller 115 is connected to the display apparatus 119 and controls display on the display apparatus 119.

The data reader/writer 116 mediates data transfer between the CPU 111 and the storage medium 120, and executes reading out of programs from the storage medium 120 and writing of the processing result of the computer 110 in the storage medium 120. The communication interface 117 mediates data transfer between the CPU 111 and another computer.

Also, specific examples of the storage medium 120 include a general-purpose semiconductor storage device such as a CF (Compact Flash (registered trademark)) and SD (Secure Digital), a magnetic storage medium such as a flexible disk, or an optical storage medium such as a CD-ROM (Compact Disk Read Only Memory).

A portion or all of the above-described embodiment can be expressed using Supplementary Notes 1 to 16, which will be described below, but there is no limitation to the following description.

(Supplementary Note 1)

A walking vibration analysis system comprising:

a vibration measurement apparatus configured to measure vibration that occurs accompanying walking of a walker;
a vibration analysis apparatus configured to extract vibration caused by contact between a heel of the walker and a floor and vibration caused by contact between a toe of the walker and the floor based on the measured vibration, and calculate a characteristic amount unique to the walker based on a result of comparing the extracted former vibration and latter vibration;
an individual specification apparatus configured to specify the walker using the calculated characteristic amount and a characteristic amount registered in advance for each individual.

(Supplementary Note 2)

The walking vibration analysis system according to Supplementary Note 1, wherein based on the result of the comparison, the vibration analysis apparatus calculates a difference between the former vibration and the latter vibration or a ratio between the former vibration and the latter vibration, as the characteristic amount.

(Supplementary Note 3)

The walking vibration analysis system according to Supplementary Note 2, wherein
the vibration analysis apparatus calculates a time difference between a start time of the vibration caused by the contact between the heel of the walker and the floor and a start time of the vibration caused by the contact between the toe of the walker and the floor, as the difference.

(Supplementary Note 4)

The walking vibration analysis system according to any one of Supplementary Notes 1 to 3, wherein
the vibration measurement apparatus calculates at least one of displacement and acceleration of a floor surface that occurs accompanying the walking as the vibration.

(Supplementary Note 5)

The walking vibration analysis system according to any one of Supplementary Notes 1 to 4, wherein
the individual specification apparatus includes:

a database in which, for each individual, a characteristic amount unique to the individual and an allowed range of error are registered in advance; and
a data comparison unit configured to specify the walker by comparing the calculated characteristic amount with the unique characteristic amount and the range of error, which were registered in advance.

(Supplementary Note 6)

The walking vibration analysis system according to Supplementary Note 5, further comprising
a data update unit configured to update the unique characteristic amount and the range of error, which are registered in the database, using the result of the comparison performed by the data comparison unit and the calculated characteristic amount,
wherein if the walker cannot be specified by the data comparison unit, the data update unit registers the reference value and the range of error of a new individual in the database using the calculated characteristic amount.

(Supplementary Note 7)

A vibration analysis apparatus, wherein
vibration caused by contact between a heel of a walker and a floor and vibration caused by contact between a toe of the walker and the floor are extracted based on vibration that occurs accompanying walking of the walker, and a characteristic amount unique to the walker is calculated based on a result of comparing the extracted former vibration and latter vibration.

(Supplementary Note 8)

A walking vibration analysis method, comprising

(a) a step of measuring vibration that occurs accompanying walking of a walker;
(b) a step of extracting vibration caused by contact between a heel of the walker and a floor and vibration caused by contact between a toe of the walker and the floor based on the measured vibration, and calculating a characteristic amount unique to the walker based on a result of comparing the extracted former vibration and latter vibration; and
(c) a step of specifying the walker using the calculated characteristic amount and a characteristic amount registered in advance for each individual.

(Supplementary Note 9)

A walking vibration analysis method according to Supplementary Note 8, wherein
in the step (b), the difference between the former vibration and the latter vibration or the ratio between the former vibration and the latter vibration is calculated as the characteristic amount based on the result of the comparison.

(Supplementary Note 10)

A walking vibration analysis method according to Supplementary Note 9, wherein
in the step (b), the time difference between the start time of vibration caused by contact between the heel of the walker and the floor and the start time of vibration caused by contact between the toe of the walker and the floor is calculated as the difference.

(Supplementary Note 11)

The walking vibration analysis method according to any one of Supplementary Notes 8 to 10, wherein
in the step (a), at least one of displacement and acceleration of a floor surface that occurs due to the walking is measured as the vibration.

(Supplementary Note 12)

The walking vibration analysis method according to any one of Supplementary Notes 8 to 11, wherein
in the step (c), the walker is specified by comparing the characteristic amount calculated in the step (b) with a characteristic amount unique to an individual and an allowed range of error, which are registered in advance for each individual.

(Supplementary Note 13)

The walking vibration analysis method according to Supplementary Note 12, further including:

(d) a step of updating the pre-registered unique characteristic amount and range of error using the result of the comparison performed in the step (c) and the calculated characteristic amount; and
(e) a step of, if the walker cannot be specified in the step (c), registering the reference value and the range of error of a new individual using the characteristic amount calculated in the step (b).

(Supplementary Note 14)

A computer-readable storage medium storing a program including commands for causing a computer to execute:

(a) a step of extracting vibration caused by contact between a heel of a walker and a floor and vibration caused by contact between a toe of the walker and the floor based on vibration that occurs accompanying walking of the walker; and
(b) a step of calculating a characteristic amount unique to the walker based on a result of comparing the extracted former vibration and latter vibration.

(Supplementary Note 15)

The computer-readable storage medium according to Supplementary Note 14, wherein

in the step (b), the difference between the former vibration and the latter vibration or the ratio between the former vibration and the latter vibration is calculated as the characteristic amount based on the result of the comparison.

(Supplementary Note 16)

The computer-readable storage medium according to Supplementary Note 15, wherein
in the step (b), the time difference between the start time of vibration caused by contact between the heel of the walker and the floor and the start time of vibration caused by contact between the toe of the walker and the floor is calculated as the difference.

Note that unless otherwise specified, the scope of the invention is not limited to only the constituent elements, types, combinations, shapes, their relative arrangements, and the like described in this embodiment, and these are merely illustrative examples.

Although the invention of the present application has been described with reference to an embodiment above, the invention of the present application is not limited to the above-described embodiment. The configurations and details of the invention of the present application can be subjected to various modifications that can be understood by a person skilled in the art within the scope of the invention of the present application.

This application claims priority based on Japanese Application No. 2016-090726 filed on April 28, 2016, the entire disclosure of which is incorporated herein.

INDUSTRIAL APPLICABILITY

Examples of using the present invention include an application of watching over the lifestyle of an elderly person or a child in a home and a security application for detecting home intrusion. Also, the present invention can be used also in an individual verification method used instead of a password, biological verification, and the like.

DESCRIPTION OF REFERENCE NUMERALS

1       Vibration measurement apparatus
2       Individual specification apparatus
3       Vibration analysis apparatus (characteristic amount extraction unit)
4       Walking vibration analysis system
11      Vibration measurement unit
12      Communication unit
21      Communication unit
22      Database
23      Data comparison unit
24      Output unit
25      Database update unit
26      Individual identifier input unit
110     Computer
111     CPU
112     Main memory
113     Storage apparatus
114     Input interface
115     Display controller
116     Data recorder/writer
117     Communication interface
118     Input device
119     Display apparatus
120     Storage medium
121     Bus

**Claims**

**1.**   A walking vibration analysis system comprising:

a vibration measurement apparatus configured to measure vibration that occurs accompanying walking of a walker;

a vibration analysis apparatus configured to extract vibration caused by contact between a heel of the walker and a floor and vibration caused by contact between a toe of the walker and the floor based on the measured vibration, and calculate a characteristic amount unique to the walker based on a result of comparing the extracted former vibration and latter vibration;

an individual specification apparatus configured to specify the walker using the calculated characteristic amount and a characteristic amount registered in advance for each individual.

2. The walking vibration analysis system according to claim 1, wherein
based on the result of the comparison, the vibration analysis apparatus calculates a difference between the former vibration and the latter vibration or a ratio between the former vibration and the latter vibration, as the characteristic amount.

3. The walking vibration analysis system according to claim 2, wherein
the vibration analysis apparatus calculates a time difference between a start time of the vibration caused by the contact between the heel of the walker and the floor and a start time of the vibration caused by the contact between the toe of the walker and the floor, as the difference.

4. The walking vibration analysis system according to any one of claims 1 to 3, wherein
the vibration measurement apparatus calculates at least one of displacement and acceleration of a floor surface that occurs accompanying the walking as the vibration.

5. The walking vibration analysis system according to any one of claims 1 to 4, wherein
the individual specification apparatus includes:

a database in which, for each individual, a characteristic amount unique to the individual and an allowed range of error are registered in advance; and
a data comparison unit configured to specify the walker by comparing the calculated characteristic amount with the unique characteristic amount and the range of error, which were registered in advance.

6. The walking vibration analysis system according to claim 5, further comprising
a data update unit configured to update the unique characteristic amount and the range of error, which are registered in the database, using the result of the comparison performed by the data comparison unit and the calculated characteristic amount,
wherein if the walker cannot be specified by the data comparison unit, the data update unit registers the reference value and the range of error of a new individual in the database using the calculated characteristic amount.

7. A vibration analysis apparatus, wherein
vibration caused by contact between a heel of a walker and a floor and vibration caused by contact between a toe of the walker and the floor are extracted based on vibration that occurs accompanying walking of the walker, and a characteristic amount unique to the walker is calculated based on a result of comparing the extracted former vibration and latter vibration.

8. A walking vibration analysis method, comprising

(a) a step of measuring vibration that occurs accompanying walking of a walker;
(b) a step of extracting vibration caused by contact between a heel of the walker and a floor and vibration caused by contact between a toe of the walker and the floor based on the measured vibration, and calculating a characteristic amount unique to the walker based on a result of comparing the extracted former vibration and latter vibration; and
(c) a step of specifying the walker using the calculated characteristic amount and a characteristic amount registered in advance for each individual.

9. A computer-readable storage medium storing a program including commands for causing a computer to execute:

(a) a step of extracting vibration caused by contact between a heel of a walker and a floor and vibration caused by contact between a toe of the walker and the floor based on vibration that occurs accompanying walking of the walker; and

(b) a step of calculating a characteristic amount unique to the walker based on a result of comparing the extracted former vibration and latter vibration.

Fig.1

WALKING VIBRATION ANALYSIS SYSTEM 4

VIBRATION MEASUREMENT APPARATUS 1

VIBRATION ANALYSIS APPARATUS 3

INDIVIDUAL SPECIFICATION APPARATUS 2

Fig.2

WALKING VIBRATION ANALYSIS SYSTEM — 4

VIBRATION MEASUREMENT APPARATUS — 1

VIBRATION MEASUREMENT UNIT — 11

VIBRATION ANALYSIS APPARATUS (CHARACTERISTIC AMOUNT EXTRACTION UNIT) — 3

COMMUNICATION UNIT — 12

INDIVIDUAL SPECIFICATION APPARATUS — 2

COMMUNICATION UNIT — 21

OUTPUT UNIT — 24

DATA COMPARISON UNIT — 23

DATABASE — 22

INDIVIDUAL IDENTIFIER INPUT UNIT — 26

DATABASE UPDATE UNIT — 25

Fig.3

Fig.4

(a)

(b)

Fig.5

| T221 | T222 | T223 | T224 |
|---|---|---|---|
| INDIVIDUAL IDENTIFIER | INDIVIDUAL WALKING VIBRATION CHARACTERISTIC AMOUNT | ERROR INFORMATION | STATISTICAL INFORMATION |
|  |  |  |  |

Fig.6

Fig.7

| j | TIME | ACCELERATION VALUE | VARIANCE VALUE | FLOOR STATE | NOTE |
|---|------|--------------------|-----------------|-------------|------|
| 0 | 0.0 | 6 | − | | |
| 1 | 0.2 | 6 | − | | |
| ... | ... | ... | ... | | |
| 397 | 79.2 | −16 | 14.4 | | |
| 398 | 79.4 | −15 | 22.1 | | |
| 399 | 79.6 | 23 | 53.7 | | |
| 400 | 79.8 | 91 | 459.2 | STILL → VIBRATING | START OF VIBRATION FOR HEEL-FLOOR CONTACT |
| 401 | 80.0 | 180 | 1936.4 | | |
| 402 | 80.2 | 293 | 5649.6 | | |
| 403 | 80.4 | 415 | 12702.9 | | |
| ... | ... | ... | ... | | |
| 723 | 144.4 | 1 | 63.2 | | |
| 724 | 144.6 | 4 | 59.6 | | |
| 725 | 144.8 | 5 | 55.3 | | |
| 726 | 145.0 | 5 | 48.3 | VIBRATING → STILL | END OF VIBRATION FOR HEEL-FLOOR CONTACT |
| 727 | 145.2 | 4 | 37.9 | | |
| 728 | 145.4 | 3 | 27.2 | | |
| 729 | 145.6 | 1 | 18.4 | | |
| ... | ... | ... | ... | | |
| 915 | 182.8 | −7 | 3.0 | | |
| 916 | 183.0 | −14 | 6.1 | | |
| 917 | 183.2 | 14 | 25.7 | | |
| 918 | 183.4 | 77 | 347.1 | STILL → VIBRATING | START OF VIBRATION FOR TOE-FLOOR CONTACT |
| 919 | 183.6 | 104 | 869.5 | | |
| 920 | 183.8 | 134 | 1654.5 | | |
| 921 | 184.0 | 177 | 2925.0 | | |
| ... | ... | ... | ... | | |
| 1097 | 219.2 | 8 | 90.5 | | |
| 1098 | 219.4 | 5 | 69.6 | | |
| 1099 | 219.6 | 3 | 56.9 | | |
| 1100 | 219.8 | 3 | 50.1 | VIBRATING → STILL | END OF VIBRATION FOR TOE-FLOOR CONTACT |
| 1101 | 220.0 | 5 | 46.9 | | |
| 1102 | 220.2 | 9 | 46.7 | | |

Fig.8

| j | TIME | ACCELERATION VALUE | VARIANCE VALUE | FLOOR STATE | NOTE |
|---|---|---|---|---|---|
| 0 | 0.0 | 1 | – | | |
| 1 | 0.2 | –1 | – | | |
| ... | ... | ... | ... | | |
| 645 | 128.8 | –2 | 1.3 | | |
| 646 | 129.0 | –2 | 1.3 | | |
| 647 | 129.2 | –2 | 1.3 | | |
| 648 | 129.4 | 127 | 780.2 | STILL → VIBRATING | START OF VIBRATION FOR HEEL–FLOOR CONTACT |
| 649 | 129.6 | 104 | 1237.2 | | |
| 650 | 129.8 | 64 | 1364.0 | | |
| 651 | 130.0 | 19 | 1357.6 | | |
| ... | ... | ... | ... | | |
| 710 | 141.8 | –5 | 135.2 | | |
| 711 | 142.0 | –3 | 86.4 | | |
| 712 | 142.2 | –3 | 60.4 | | |
| 713 | 142.4 | –4 | 53.6 | VIBRATING → STILL | END OF VIBRATION FOR HEEL–FLOOR CONTACT |
| 714 | 142.6 | –5 | 53.8 | | |
| 715 | 142.8 | –4 | 47.3 | | |
| 716 | 143.0 | –5 | 26.8 | | |
| ... | ... | ... | ... | | |
| 997 | 199.2 | 6 | 2.3 | | |
| 998 | 199.4 | 6 | 2.6 | | |
| 999 | 199.6 | 7 | 3.3 | | |
| 1000 | 199.8 | 177 | 1436.1 | STILL → VIBRATING | START OF VIBRATION FOR TOE–FLOOR CONTACT |
| 1001 | 200.0 | 201 | 3116.1 | | |
| 1002 | 200.2 | 298 | 6685.9 | | |
| 1003 | 200.4 | 246 | 8673.9 | | |
| ... | ... | ... | ... | | |
| 1099 | 219.6 | –5 | 65.4 | | |
| 1100 | 219.8 | –5 | 64.5 | | |
| 1101 | 220.0 | –5 | 56.8 | | |
| 1102 | 220.2 | –4 | 44.5 | VIBRATING → STILL | END OF VIBRATION FOR TOE–FLOOR CONTACT |
| 1103 | 220.4 | –3 | 29.5 | | |
| 1104 | 220.6 | –4 | 17.7 | | |
| 1105 | 220.8 | –5 | 11.7 | | |

Fig.9

(a)

START OF VIBRATION
DURING HEEL-FLOOR
CONTACT

START OF VIBRATION
DURING TOE-FLOOR
CONTACT

ACCELERATION

TIME

END OF VIBRATION
DURING HEEL-
FLOOR CONTACT

END OF VIBRATION
DURING TOE-
FLOOR CONTACT

(b)

START OF VIBRATION
DURING HEEL-FLOOR
CONTACT

START OF VIBRATION
DURING TOE-FLOOR
CONTACT

ACCELERATION

TIME

END OF VIBRATION
DURING HEEL-
FLOOR CONTACT

END OF VIBRATION
DURING TOE-
FLOOR CONTACT

26

Fig.10

```
        ┌─────────────┐
        │    Start     │
        └──────┬───────┘
               │
               ▼
    ┌────────────────────────┐
    │ ACQUIRE WALKING VIBRATION │──── S21
    │  CHARACTERISTIC AMOUNT   │
    └───────────┬────────────┘
                │
                ▼
    ┌────────────────────────┐
    │  COMPARE ACQUIRED WALKING │──── S22
    │  VIBRATION CHARACTERISTIC │
    │   AMOUNT WITH DATABASE   │
    └───────────┬────────────┘
                │              S23
                ▼
         ◇─────────────◇
        ╱  MATCHES WITH  ╲        No
       ╱ WALKING VIBRATION ╲───────────────┐
       ╲   CHARACTERISTIC  ╱                │
        ╲ AMOUNT IN DATABASE? ╱             │
         ◇─────────────◇                    │
               │ Yes                        │
               ▼                            │
    ┌────────────────────────┐              │
    │     SPECIFY WALKER      │──── S24      │
    └───────────┬────────────┘              │
                │                           │
                ▼                           │
    ┌────────────────────────┐              │
    │  OUTPUT COMPARISON RESULT │──── S25    │
    └───────────┬────────────┘              │
                │         S26          S27  │
                ▼                           ▼
    ┌────────────────────────┐   ┌──────────────────────┐
    │  RE-CALCULATE AND UPDATE │   │  REGISTER NEW RECORD  │
    │    WALKING VIBRATION     │   └──────────┬───────────┘
    │ CHARACTERISTIC AMOUNT, ERROR │          │
    │ INFORMATION, AND STATISTICAL │          │
    │       INFORMATION        │             │
    └───────────┬────────────┘              │
                │◄───────────────────────────┘
                ▼
        ┌─────────────┐
        │     End      │
        └─────────────┘
```

Fig.11

| INDIVIDUAL IDENTIFIER | INDIVIDUAL WALKING VIBRATION CHARACTERISTIC AMOUNT | ERROR INFORMATION | STATISTICAL INFORMATION | |
|---|---|---|---|---|
| USER ID | AVERAGE VALUE (MILLISECONDS) | VARIANCE VALUE | SAMPLE COUNT | SQUARED SUM TOTAL |
| USER A | 65.2 | 7.8 | 9 | 38261.4 |
| USER B | 88.4 | 4.1 | 12 | 93776.0 |
| USER C | 105.0 | 6.6 | 10 | 110316.0 |
| USER D | 133.3 | 5.6 | 16 | 284308.9 |
| ... | ... | ... | ... | ... |

Fig.12

| INDIVIDUAL IDENTIFIER | INDIVIDUAL WALKING VIBRATION CHARACTERISTIC AMOUNT | ERROR INFORMATION | STATISTICAL INFORMATION | |
|---|---|---|---|---|
| USER ID | AVERAGE VALUE (MILLISECONDS) | VARIANCE VALUE | SAMPLE COUNT | SQUARED SUM TOTAL |
| USER A | 65.2 | 7.8 | 9 | 38261.4 |
| USER B | 88.4 | 4.1 | 12 | 93776.0 |
| USER C | 104.9 | 6.2 | 11 | 121049.0 |
| USER D | 133.3 | 5.6 | 16 | 284308.9 |
| ... | ... | ... | ... | ... |

Fig.13

4

**WALKING VIBRATION ANALYSIS SYSTEM**

1

**VIBRATION MEASUREMENT APPARATUS**

11

VIBRATION
MEASUREMENT
UNIT

12

COMMUNICATION
UNIT

2

**INDIVIDUAL SPECIFICATION
APPARATUS**

21

COMMUNICATION
UNIT

3

VIBRATION ANALYSIS
APPARATUS
(CHARACTERISTIC AMOUNT
EXTRACTION UNIT)

24

OUTPUT UNIT

23

DATA
COMPARISON
UNIT

22

DATABASE

26

INDIVIDUAL
IDENTIFIER
INPUT UNIT

25

DATABASE
UPDATE UNIT

Fig.14

110
COMPUTER

| 111 | 112 | 113 |
|---|---|---|
| CPU | MAIN MEMORY | STORAGE DEVICE |

121

| 114 | 115 | 116 | 117 |
|---|---|---|---|
| INPUT INTERFACE | DISPLAY CONTROLLER | DATA READER/WRITER | COMMUNICATION INTERFACE |

| INPUT DEVICE | DISPLAY DEVICE | RECORDING MEDIUM |
|---|---|---|

118　　　　　119　　　　　120

OTHER COMPUTER, ETC.

## INTERNATIONAL SEARCH REPORT

| | International application No. |
|---|---|
| | PCT/JP2017/016867 |

**A.   CLASSIFICATION OF SUBJECT MATTER**
*A61B5/117*(2016.01)i,  *A61B5/11*(2006.01)i,  *G08B13/02*(2006.01)i,  *G08B25/04*
(2006.01)i

According to International Patent Classification (IPC) or to both national classification and IPC

**B.   FIELDS SEARCHED**

Minimum documentation searched (classification system followed by classification symbols)
A61B5/117, A61B5/11, G08B13/02, G08B25/04

Documentation searched other than minimum documentation to the extent that such documents are included in the fields searched
Jitsuyo Shinan Koho          1922–1996   Jitsuyo Shinan Toroku Koho   1996–2017
Kokai Jitsuyo Shinan Koho    1971–2017   Toroku Jitsuyo Shinan Koho   1994–2017

Electronic data base consulted during the international search (name of data base and, where practicable, search terms used)

**C.   DOCUMENTS CONSIDERED TO BE RELEVANT**

| Category* | Citation of document, with indication, where appropriate, of the relevant passages | Relevant to claim No. |
|---|---|---|
| X | JP 2004-227053 A  (Yamatake Corp.), 12 August 2004 (12.08.2004), paragraph [0033] (Family: none) | 1-9 |
| X | JP 2007-319347 A  (Anima Corp.), 13 December 2007 (13.12.2007), paragraphs [0003] to [0004] (Family: none) | 7,9 |
| A | JP 2002-197437 A  (Sony Corp.), 12 July 2002 (12.07.2002), paragraphs [0018] to [0057] & US 2002/0107649 A1 paragraphs [0049] to [0154] | 1-9 |

☐   Further documents are listed in the continuation of Box C.          ☐   See patent family annex.

| | |
|---|---|
| *       Special categories of cited documents: | "T"   later document published after the international filing date or priority date and not in conflict with the application but cited to understand the principle or theory underlying the invention |
| "A"   document defining the general state of the art which is not considered    to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 04 July 2017 (04.07.17) | 18 July 2017 (18.07.17) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

## REFERENCES CITED IN THE DESCRIPTION

**Patent documents cited in the description**

- JP 2004227053 A
- JP 2007304955 A
- JP 2016090726 A

**Non-patent literature cited in the description**

- **HIDEO WATANABE ; AKIHIKO MATSUOKA ; SHO KIMURA ; KATSUO INOUE.** Vibration Response Characteristics in Residential Floor Finish Structure by Walking - Study of the residential floor vibration characteristics and floor impact sound insulation performance taking footstep sensation into consideration, Part 3. Toda Corporation, 1998
- **TETSUO AKIYAMA ; RYO MURATA ; KEITA MIZUNO ; NOBORU SEKIYA.** Relationship Between Step Interval, Walking Pattern, and Energy Consumption Amount in a Healthy Person. *Congress of the Japanese Physical Therapy Association,* 2009